# EUROPEAN PATENT SPECIFICATION

(11) **EP 3 777 675 B1**
(45) Date of publication and mention of the grant of the patent: **07.06.2023**
(21) Application number: 19191219.5
(22) Date of filing: 12.08.2019
(51) Int. Cl.: A61B 5/053, A61B 5/00, A61B 5/01

(54) **WEARABLE SENSOR DEVICE**
WEARABLE-SENSORVORRICHTUNG
DISPOSITIF DE CAPTEUR PORTABLE

(43) Date of publication of application: 17.02.2021
(73) Proprietor: Onera Technologies B.V., 5617 BD Eindhoven (NL)
(72) Inventor: Ermers, Petrus Wilhelmus Johannes, 5617 BD Eindhoven (NL); Borm, Martina Adriana Wilhelmina, 5617 BD Eindhoven (NL)
(74) Representative: AWA Sweden AB

(56) References cited:
- EP-A1- 2 589 333
- EP-B1- 2 948 054
- US-A1- 2014 051 946
- US-A1- 2019 231 199
- US-A1- 2019 231 199

## Description

### Technical Field

The present disclosure relates to a wearable sensor device, and in particular a wearable sensor device for acquisition of physiological data of a subject by means of electrodes.

### Background

Acquisition of physiological data via a subject's skin is conventionally done by means of electrodes, wherein the electrodes are connected to an electronics module in a recorder or playback equipment using conductive wires. However, there are quite a few disadvantages with this setup. Since the distances between each of the wires are not fixed, they can vary in such a way so that they electromagnetically interfere with each other or with devices in the environment in an unpredictable way, thereby possibly causing artefacts to appear in the measured signals. Moreover, the wrong physiological signals could possibly be picked up. There is also the potential risk for dislodgement of the electrodes by accidental pulling of the wires. Moreover, the procedure of connecting the wires can also be time consuming, and the plurality of wires may hinder the systems' usability since it limits the movement of the user and also introduces the risk of connecting the wires in an erroneous way.

One way of solving the issue associated with the use of wires is to use an adhesive patch incorporating said plurality of electrodes, and mounting an electronics module onto the adhesive patch for controlling the acquisition of physiological data. However, in this setup the measured signals containing information of physiological data must travel through a base structure of the patch to get from the electrodes to the electronics module. This comes with the disadvantage of having to twist the conductive tracks, often by 90 - 180 degrees, into the electronics module, thereby introducing an increased mechanical stress in a part of the electrode track. Moreover, this elongation of the conductive tracks may increase the electrode path, thereby increasing the risk of a loss in signal quality. A further disadvantage is that in order for certain types of sensors to be used to conduct measurements, for example, optical sensors, the patch must incorporate openings exposing the skin beneath the patch so as to provide line of sight to the user's skin for said optical sensors. This may increase manufacturing cost of the patch, and also weaken the structural integrity of the patch.

US 2014/051946 A1 discloses one patch solution of a wearable sensor device. Further patch solutions are i.e. disclosed in US 2019/231199 A1 and EP 2 589 333 A1.

Hence, there is a need for an improved solution that alleviates at least some of the mentioned drawbacks with present solutions, and in particular a wearable sensor device for comfortable wear.

### Summary

It is an object of the present invention to provide an improved solution that alleviates the mentioned drawbacks with present solutions.

According to a first aspect of the invention, a wearable sensor device for arrangement on a skin surface of a subject according to claim 1 is provided.

The base structure of the wearable sensor device provides a first bottom surface and a second bottom surface, which are facing the skin, wherein the first skin facing surface is located closer to a skin surface than the second skin facing surface, when the wearable sensor device is arranged to the subjects' skin. When in use, the first skin facing surface may be in contact with the skin of the subject, and the second skin facing surface may be at a distance from the skin of the subject.

Also, in the following, a bottom side, a bottom surface, a bottom portion etc. may refer to a side, a surface or a portion of the wearable sensor device, or a side, a surface or a portion of any of its components, facing toward the skin surface of the subject when the wearable sensor device is arranged thereto. Likewise, a top side, a top surface, a top portion etc. may refer to a side, a surface, a portion of the wearable sensor device, or a side, a surface, or a portion of any of its components, facing away from the skin of a subject when the wearable sensor device is arranged thereto.

Hence, the proximal skin facing surface may be a proximal bottom surface. The distal skin facing surface may be a distal bottom surface.

By this wearable sensor device, the electronics module is arranged such that, when the wearable sensor device is arranged to a skin surface of a subject, it resides between the base structure and the subject's skin. Thereby, the electrodes and the electronics module are arranged on the same side of the base structure. This presents the advantage of not having to twist the conductive tracks through the base structure in order to connect the electrodes to the electronics module, and in particular the often 90 - 180 degrees common in present solutions. Instead, the electrically conductive tracks may be adapted to extend along a skin facing surface of the basic structure between the electrodes and the electronics module, without significant twisting of the electrically conductive tracks. Hence, the electrically conductive tracks are subjected to less mechanical stress as compared to conductive tracks of present solutions. Moreover, since the degree of twisting the electrically conductive tracks is reduced, an increase in electrode path is prevented, which in turn may prevent significant losses of signal quality.

Moreover, since the electronics module is arranged on a skin facing side of the base structure, it is inherently protected by the base structure when the wearable sensor device is arranged on the subject's skin. Hence, a user may be prevented from accidentally dislodging the electronics module and the electrodes from one another. In addition, since there are no wires hanging about, the movement of a user is not impaired in any significant way while wearing the wearable sensor device.

A further advantage of this invention is that the base structure need no through-holes in order to establish connection between the electrodes and the electronics module. Hence, the structural integrity of the base structure may be increased or preserved.

The proximal skin facing surface may be adapted to be at least partly in contact with a subjects' skin during use. The wearable sensor device may be adapted to be fixated to the skin surface of the subject by means of the proximal skin facing surface. The proximal skin facing surface may be adapted to provide an adhesive surface by which the wearable sensor device attaches to the skin surface. Alternatively, the wearable sensor device may be fixated to a skin surface of a subject by means of a strap.

The distal skin facing surface may be adapted to be at least partly in contact with the electronics module. The distal skin facing surface may be a surface covering a top surface of the electronics module. In one embodiment, the entire top surface of the electronics module may be covered by the distal skin facing surface.

The elevation distance between the proximal skin facing surface and the distal skin facing surface, when in use, may correspond to the height of the electronics module. The height of the electronics module may be the distance from the bottom surface to the top surface of the electronics module. The height of the electronics module may be e.g., 0,5 cm - 2 cm.

In addition, the wearable sensor device may be a single- or multi- sensor device. By single sensor device, it may be meant that the wearable sensor device incorporates a single type of sensors, such as electrodes. By multi sensor device, it may be meant that the wearable sensor device incorporates a plurality of sensors of different types, such as electrodes and optical sensors.

The electrically conductive tracks may be adapted to transmit electrical signals provided by the electrodes to the electronics module. The electrically conductive tracks may be adapted to transmit one or more source electrical signals to the electrodes for transmission into a subject's tissue, wherein a response of the one or more source electrical signals are measured by the electrodes. The electronics module may comprise sensors adapted to provide data of a subject. The electronics module may be adapted to receive data from sensors of the electronics module. The wearable sensor device may be adapted such that the electronics module remains in a stable position for the duration of monitoring.

The relative positioning of the electronics module to the base structure may be such that, besides collection of physiological data by means of the electrodes, physiological data can also be collected directly from the users' tissue to the electronics module. In other words, direct contact between, or at least an unobstructed line of sight from, the electronics module and a subject's skin may be established. Hence, the electronics module may incorporate a greater variety of sensors, for instance optical sensor.

In addition, the wearable sensor device may be a non-invasive active device intended for diagnosis and/or monitoring for continuous short-term usage. By short term usage, it may be meant a time duration between a couple of minutes, for instance 60 minutes, up to a couple of days, for instance 30 days.

The wearable sensor device may be applied to a user at pre-defined areas on the users' skin, depending on the nature of the monitoring. It may for instance be applied on the torso of a user. It may for instance be applied on the arms or legs of a user. It may for instance be applied specifically to the chest of a user. It may for instance be applied specifically to the stomach of a user. It may for instance be applied specifically to the forehead of a user. By applying it to the forehead of a user, more information may be extracted, for example EEG- and EOG-signals.

The wearable sensor device may comprise two, three, four, five, six, seven, eight, nine, ten, or more electrodes. The wearable sensor device may comprise a two, three, four, five, six, seven, eight, nine, ten or more electrically conductive tracks. The electrically conductive tracks may be arranged such that they are not electrically connected to each other. The electrically conductive tracks may be electrically isolated from each other.

According to one embodiment, the proximal skin facing surface may be adapted to extend at least partly along a circumference of the distal skin facing surface, preferably 50% - 100%. The proximal skin facing surface may be adapted to extend fully along a circumference of the distal skin facing surface. The outward extent of the proximal skin facing surface away from the region of the distal skin facing surface may be such that it provides a moisture sealing together with a subjects' skin, so that outside moisture cannot penetrate into the wearable sensor device. The wearable sensor device may be adapted such that the moisture sealing is formed along the whole circumference of a skin-contact edge of the wearable sensor device.

According to one embodiment, the proximal skin facing surface and the distal skin facing surface may be at least partly joined by an inclined surface along which said electrically conductive tracks extends between the proximal skin facing surface and the distal skin facing surface. The inclined surface may be formed partly by a portion of the proximal skin facing surface and/or a portion of the distal skin facing surface. Alternatively, the inclined surface may be constituted by an interface surface arranged in-between the proximal skin facing surface and the distal skin facing surface. The boundary between the proximal skin facing surface and the distal skin facing surface may be clearly defined by a transversal edge extending between the two, which edge may be interrupted piecewise or extending continuously. By the inclined surface, the mechanical stress of the electrically conductive tracks may be further reduced.

According to one embodiment, the distal skin facing surface may be located in a recess in the base structure. By this, the elevation difference between a bottom side of an electronics module and the plurality of electrodes may be decreased, thereby reducing the degree of twisting of the electrically conductive tracks needed in order to connect the plurality of electrodes to the electronics module.

According to one embodiment, the base structure may be made from a flexible material. By this, the base structure may be bent around the electronics module when the wearable sensor device is arranged to a skin surface of a subject. The proximal skin facing surface and the distal skin facing surface may be provided by bending the base structure around the electronics module.

The bending of the base structure around the electronics module may cause the electrically conductive tracks to curve. The local curvature C at an arbitrarily point P located on any of the electrically conductive tracks may be expressed as C(P) = 1/R, i.e. in terms of the curvature of a circle, having radius R, which approximates the curvature near point P. The proximal skin facing surface and the distal skin facing surface may be provided, when in use, by the flexible material of the base structure such that the curvature C of the electrically conductive tracks at a point P along any of the electrically conductive tracks does not exceed 1 cm⁻¹, preferably does not exceed 0,5 cm⁻¹, and most preferably does not exceed 0,25 cm⁻¹. By this, the electrically conductive tracks may be prevented from being subjected to a magnitude of mechanical stress which may damage the electrically conductive tracks.

According to one embodiment, the base structure may comprise two or more proximal skin facing surface portions separated from each other, wherein at least one electrode is arranged on each of the two or more proximal skin facing surface portions. By this, the distance between at least two electrodes may be increased, while still being protected by the base structure. Two of said two or more proximal skin facing surface portions may be arranged on opposite sides of the distal skin facing surface. The base structure may comprise three, four, five, six or more proximal skin facing surface portions. The proximal skin facing surface portions may be arranged such that they surround the distal skin facing surface.

According to one embodiment, the wearable sensor device may comprise two electrodes arranged on a single proximal skin facing surface portion. By this, physiological data of a subject may be measured using a greater variation of measurement configurations. For instance, a bio potential may be measured in-between the two electrodes arranged on the single proximal skin facing surface portion. Alternatively, a bio potential may be measured in-between two electrodes arranged on separate proximal skin facing surface portions.

According to one embodiment, one or more electrodes may be arranged directly on the proximal skin facing surface. Alternatively, the one or more electrodes are arranged on the proximal skin facing surface such that a protective layer reside there in-between. The protective layer may prevent at least a portion of the tracks material from making direct contact with the skin tissue. The protective layer may be of a dielectric material.

According to one embodiment, the electrically conductive tracks may comprise contact terminals adapted to be in contact with corresponding contact terminals on the electronics module. By this, the manufacturing process may be facilitated, leading to quicker manufacturing process and/or cheaper manufacturing process. The contact terminals of the electrically conductive tracks may be shaped as discs, squares, rectangles etc., and the contact terminals of the electronics module may have a corresponding shape.

According to one embodiment, the electronics module may be arranged on the terminal contacts of the electrically conductive tracks. The terminal contacts of the electronics module may be arranged along a top surface of the electronics module. By this, an easier assembling of the components may be enabled. Moreover, the terminal contacts may be protected in-between the base structure and the electronics module. Also, by this, the electronics module and the electrically conductive tracks may be less likely to be dislodged from each other.

According to one embodiment, the electrically conductive tracks between the electrodes and the contacts may be covered by a dielectric. By this, a leakage current may be prevented from the electrically conductive tracks, thereby reducing the degree of artefacts in collected physiological data.

According to one embodiment, the contacts terminal of the electrically conductive tracks may be arranged adjacent to each other in a grid formation on the distal skin facing surface. By adjacent, it may be meant that there is a predetermined separation distance between each contact terminal.

According to one embodiment, the electrically conductive tracks extend out from the contact terminals toward the electrodes first in a lateral direction of the base structure and then in a longitudinal direction of the base structure. Alternatively, the electrically conductive tracks may extend out from the contact terminals toward the electrodes first in a longitudinal direction of the base structure and then in a lateral direction of the base structure. By this, two electrodes of a single skin facing surface portion may be more separated more in a lateral direction of a base structure for a predetermined lateral extension of the electronics module.

According to one embodiment, the electronics module may comprise one or more sensors arranged such that they face the skin surface when the wearable sensor device is arranged thereto.

According to one embodiment, at least one of the one or more sensors is an optical sensor.

According to one embodiment, the one or more sensors of the electronics module may be in direct contact with the skin surface when the wearable sensor device is arranged thereto

According to one embodiment, a top portion of the electronics module may be dome shaped. By this, a contact surface between the top portion of the electronics module and the distal skin facing surface may be increased. Moreover, by a dome shaped top portion, it may provide improved support to the electrically conductive tracks. The top portion of the electronics module may be flat. In addition, a top portion of the dome-shaped electronics module may be flat.

According to one embodiment, the electronics module may comprise a printed circuit board, sensory electronics and a battery. The electronics module may comprise a wireless receiving transmission unit, WRTU. The WRTU may communicate wirelessly by means of e.g. wireless local area network, WLAN; near-field communication, NFC; or Bluetooth or Bluetooth Low Energy. The electronics module may comprise other sensors, such as electrochemical sensors, optical sensors, electronic sensors, piezoelectric sensors, gravimetric sensors, or pyroelectric sensors. The electronics module may comprise a temperature sensor configured to measure the temperature of the skin surface of the subject.

The electronics module may comprise an amplifier configured to amplify a measured electrical signal of the electrodes. The electronics module may comprise a filtering unit configured to filter the measured electrical signals in terms of noise.

According to one embodiment, the wearable sensor device may be adapted to be fixed to a skin surface by means of an adhesive. The adhesive may be provided in the form of an adhesive layer arranged on at least a part of the bottom side of the base structure.

According to one embodiment, the base structure may be made of a biocompatible material. By biocompatible, it may be meant not having any toxic or injurious effect on a skin surface during contact. The base structure may comprise a plurality of layers having different properties. The base structure layer located closest to the skin surface when in use may be of a biocompatible material.

According to one embodiment, the wearable sensor device may be adapted to be reusable. By this, the adhesive layer may for example be removed and replaced by a new adhesive layer, thereby allowing for the wearable sensor device to be arranged to a skin surface of a subject once more.

According to a second aspect of the invention, a method of manufacturing a wearable sensor device according to the first aspect of the invention, or any embodiments thereof, is provided according to the independent claim 14.

According to a third aspect of the invention, a use of the wearable sensor device according to the first aspect of the invention, or any embodiments thereof, to measure a bio-impedance signal of a subject, is provided according to the independent claim 15.

In one embodiment, the wearable sensor device may be used to that it is applied to a user at pre-defined areas on the users' skin, depending on the nature of the monitoring that is to be taking place. In one embodiment, it may be used so that it is applied on the torso of a user. In one embodiment, it may be used so that it is applied on the arms or legs of a user. In one embodiment, it may be used so that it is applied to the chest of a user. In one embodiment, it may be used so that it is applied to the stomach of a user. In one embodiment, it may be used so that it is applied specifically to the forehead of a user. By applying it to the forehead of a user, more information may be extracted, for example EEG- and EOG-signals. In one embodiment, the wearable sensor device may be used by a user during his or her everyday life, for instance at home or at work, away from the location at where the wearable sensor device was applied, for instance at a medical facility.

The invention is defined by the appended independent claims, with embodiments being set forth in the appended dependent claims, in the following description and in the drawings.

### Brief Description of the Drawings

The invention will in the following be described in more detail with reference to the enclosed drawings, wherein:
Fig. 1a illustrates a schematic view of a wearable sensor device according to a prior art solution;
Fig. 1b illustrates a schematic view of a wearable sensor device according to a prior art solution;
Fig. 2 illustrates a perspective view of a wearable sensor device according to an embodiment of the present invention;
Fig. 3a illustrates an exploded view of a wearable sensor device according to an embodiment of the present invention, as seen from below;
Fig. 3b illustrates an exploded view of a wearable sensor device according to an embodiment of the present invention, as seen from above;
Fig. 4a illustrates a schematic view of a wearable sensor device according to an embodiment of the present invention;
Fig. 4b illustrates a schematic view of a wearable sensor device according to an embodiment of the present invention;
Fig. 5 illustrates a flow chart of a method according to an embodiment of the present invention.

### Description of Embodiments

The present invention will be described more fully hereinafter with reference to the accompanying drawings, in which preferred embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art. In the drawings, like numbers refer to like elements.

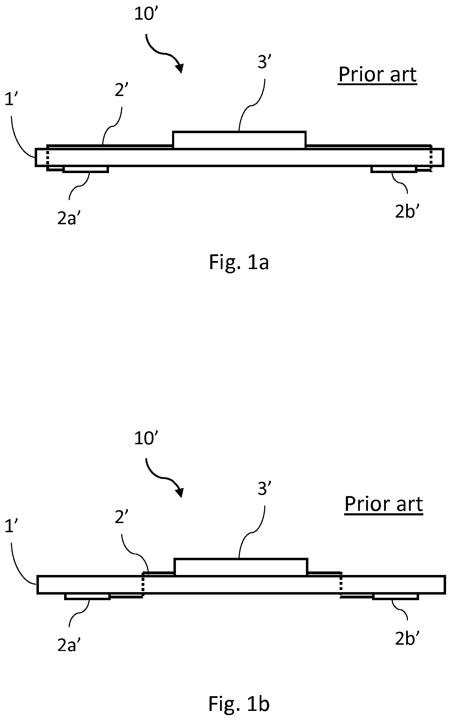

In the prior art, the conventional way of connecting electrodes 2a', 2b' in a wearable sensor device 10' to an electronics module 3' is to pull the electrically conductive tracks 2' through a base structure 1' of the wearable sensor device 10' as shown in Fig. 1a and Fig. 1b. In both cases, the electrically conductive tracks 2' are twisted into the base structure 1', and then twisted so that they align with the top surface of the base structure 1' before being connected to the electronics module 3'. In the first case, as depicted in Fig. 1a, the electrically conductive tracks 2' are twisted about 180 degrees in order to align with the top surface of the base structure 1'. In the second case, as depicted in Fig. 1b, the electrically conductive tracks are twisted 90 degrees into the base structure 1' and then twisted 90 degrees into a tangential direction of the top surface of the base structure 1'. Due to this significant twisting of the electrically conductive tracks, an increased mechanical stress is introduced in the twisted parts of the electrically conductive tracks. Also, the electrically conductive tracks 2' are elongated which increases the electrode path, which consequently increases the risk of a loss in signal quality.

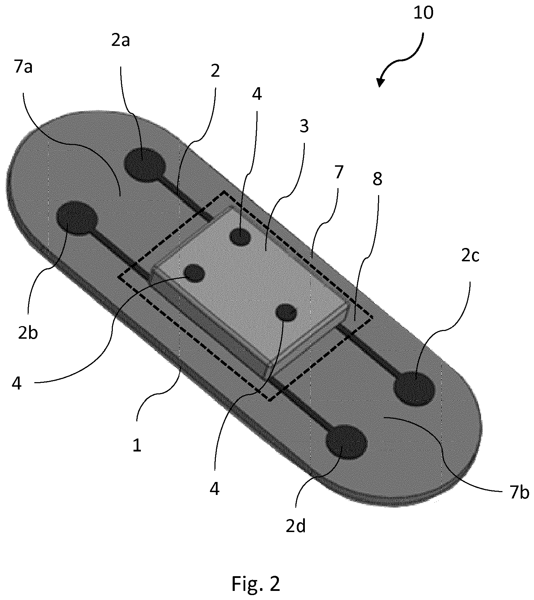

Fig. 2 illustrates a perspective view of a wearable sensor device 10 according to an embodiment of the present invention. According to one embodiment, the wearable sensor device 10 comprises a base structure 1 which, when the wearable sensor device 10 is arranged to a skin surface 100 of a subject, provides a proximal skin facing surface 7 proximal to the skin surface 100 and a distal skin facing surface 8 distal to the skin surface 100. More specifically, the base structure 1 comprises a flat and elongated shape, wherein the two ends of the base structure provide a first and second proximal skin facing surfaces 7a, 7b. Together, they constitute a portion of the proximal skin facing surface 7. The first and the second proximal skin facing portions 7a, 7b are arranged on opposite sides of the distal skin facing surface 8. The remainder of the proximal skin facing surface 7 is constituted by two skin facing side surfaces which connect the first and the second proximal skin facing portions 7a, 7b along opposite sides of the distal skin facing surface 8.

On each proximal skin facing surface portion 7a, 7b, two electrodes are arranged. On the first skin facing surface portion 7a, a first and a second electrode 2a, 2b is located. On the second skin facing surface portion 7b, a third and a fourth electrode 2c, 2d is located. The electrodes 2a, 2b, 2c, 2d are configured to, when in use, provide an electrical signal containing information of the subject. Moreover, the wearable sensor device 10 comprises an electronics module 3 arranged on the distal skin facing surface 8, which is configured to process the electrical signal provided by the electrodes 2a, 2b, 2c, 2d. The first electrode 2a, the second electrode 2b, the third electrode 2c, and the fourth electrode 2d are electrically connected to the electronics module 3 by means of separate electrically conductive tracks 2. The electrically conductive tracks 2 are separated from each other.

The base structure 1 has the function of providing a bottom surface onto which the electrodes, the conductive tracks and the electronics module can be fixated. When correctly arranged on a skin surface 100 of a subject, the electronics module 3 is arranged in-between the subject's skin surface 100 and the base structure 1. Hence, the electrically conductive tracks 2 do not need to be twisted through the base structure in order to be electrically connected to the electronics module 3. Rather, the electrically conductive tracks 2 merely extend along the proximal skin facing surface 7 and the distal skin facing surface 8, thereby reducing the mechanical stress of the electrically conductive tracks 2 during use.

Moreover, since the electronics module 3 is arranged on the same side of the base structure 1 as the electrically conductive tracks 2, it is possible to have a direct interconnect system between these two elements without having to significantly twist the electrode tracks 2. The relative positioning of the electronics module is such that next to the collection of physiological data from the electrodes, physiological data can also be collected directly from the user's tissue at the skin surface by sensors 4 arranged on the bottom surface of the electronics module 3. The sensors 4 may for instance be optical sensors or temperature sensors.

The wearable sensor device 10 of the present invention enables all components, i.e. the electrodes 2a, 2b, 2c, 2d, the electrically conductive tracks 2, the electronics module 3, to be held in a secured position and simultaneously achieve smaller distances and less curvatures in the path of the electrical signals. This enables to possibility of obtaining reliable signals of e.g. bio-potential while improving on usability as compared to existing solutions.

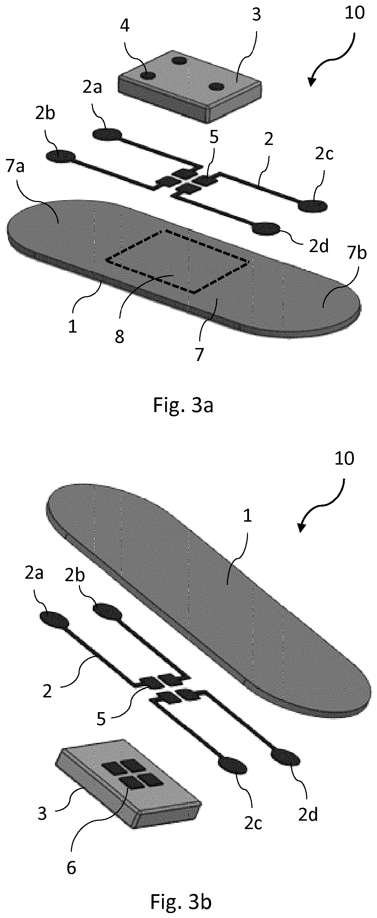

Figs. 3a and 3b illustrate an exploded view of a wearable sensor device according to an embodiment of the present invention, as seen from above and below, respectively. Each of the four electrically conductive tracks 2 comprises a contact terminal 5 which is configured to electrically connect to a corresponding contact terminal 6 arranged on a top surface of the electronics module 3. The contact terminals 5 of the electrically conductive tracks 2 are arranged in a grid formation, and merely separated a small distance from each other. Moreover, the electrically conductive tracks 2 extend out from the contact terminals 5 first in a lateral direction and then extend in a longitudinal direction out of the distal skin facing surface 8.

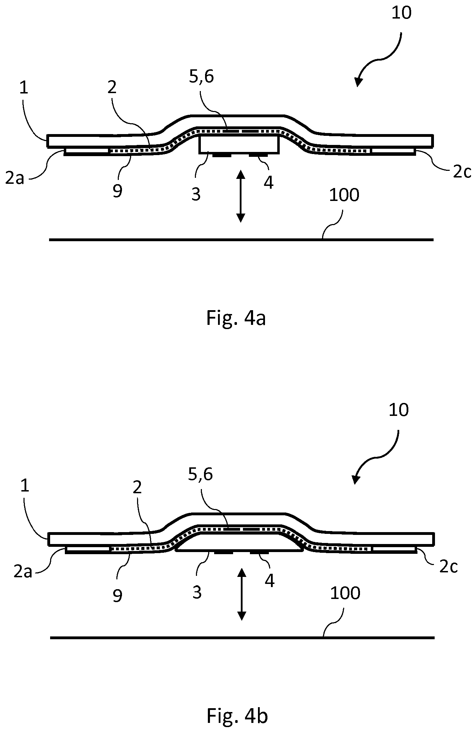

In one embodiment, the electrically conductive tracks 2 are covered by a dielectric material 9, see Figs. 4a, 4b. The dielectric material may be provided as a layer covering the electrically conductive tracks 2 on both the proximal skin facing surface 7 and the distal skin facing surface 8. The dielectric layer may cover the entire or a major part of the bottom surface of the base structure 1 except the electrodes 2a, 2b, 2c, 2d and the contact terminals 5, i.e. the entire or a major part of the proximal skin facing surface 7 and the distal skin facing surface 8. It may specifically cover the electrically conductive tracks 2. The dielectric material 9 may cover the electrically conductive tracks 2 while not covering large portions of the skin facing surface 7 and/or the distal skin facing surface 8.

Fig. 4a and 4b illustrate schematic views of a wearable sensor device according to an embodiment of the present invention. In Fig. 4a, the wearable sensor device 10 is depicted in a mounting shape but displaced from the skin surface 100 of a subject. The base structure 1, having a flexible material, bends around the electronics module 3, which in this particular embodiment has a cuboid shape. The contact terminals 5 of the electrically conductive tracks 2 are electrically connected to the contact terminals 6 located on the top surface of the electronics module 3. On each side of the electronics module 3, the electrically conductive tracks 2 curves in accordance with the curvature of the flexible base structure 1. As can be seen in Fig. 4a, there is a gap between longitudinal sides of the electronics module 3 and the base structure 1.

In Fig. 4b, the wearable sensor device 10 is also depicted in a mounting shape but displaced from the skin surface 100 of a subject. However, contrary to the wearable sensor device 10 depicted in Fig. 4a, the wearable sensor device 10 depicted in Fig. 4b comprises a dome-shaped electronics module 3 with a flat top surface. As can be seen in Fig. 4b, the gap between longitudinal sides of the electronics module 3 and the base structure 1 has been significantly reduced.

Moreover, as can be seen in Figs. 4a and 4b, the electronics module 3 comprises sensors located on its bottom side. These may be brought into direct contact with the skin surface of a subject when the wearable sensor device is arranged thereto. Thus, sensors 4 which require direct contact or at least unobstructed line of sight may be incorporated into the wearable sensor device. For instance, such sensors may be optical sensors or temperature sensors.

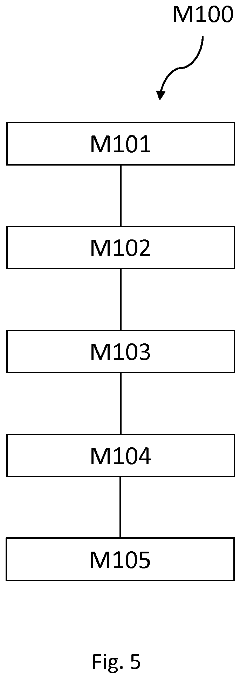

Fig. 5 illustrates a flow chart of a method M100 according to an embodiment of the present invention. The method M100 comprises the steps of: providing M101 a base structure 1; arranging M102 electrically conductive tracks 2 such that they extend from the electrodes 2a, 2b, 2c, 2d to a distal skin facing surface 8; arranging M103 one or more electrodes 2a, 2b, 2c, 2d on a proximal skin facing surface 7 of the base structure 1; providing M104 an electronics module 3 comprising one or more contacts 6; and arranging M105 the electronics module to the base structure 1 such that the one or more contact terminals 6 on the electronics module 3 are in contact with the one or more contact terminals 5 of the electrically conductive tracks.

By this, a simplification in production process regarding the establishment of an interconnection between the electronics module and the conductive tracks is established, since no additional features need to be added for the electrical signal to reach the electronics module through the base structure 1.

In the drawings and specification, there have been disclosed preferred embodiments and examples of the invention and, although specific terms are employed, they are used in a generic and descriptive sense only and not for the purpose of limitation, and may be combined in various ways unless being clearly incompatible with one another. However, the scope of the invention is set forth in the following claims.

## Claims

1. A wearable sensor device (10) for arrangement to a skin surface (100) of a subject, comprising
a base structure (1) which, when the wearable sensor device (10) is arranged to a skin surface (100) of a subject, provides a first bottom surface (7) and a second bottom surface (8), wherein the first bottom surface (7) is located closer to a skin surface (100) than the second bottom surface (8) when the wearable sensor device (10) is arranged to a skin surface (100) of a subject;
one or more electrodes (2a, 2b, 2c, 2d) arranged on the first bottom surface (7) of the base structure (1) and configured to provide an electrical signal containing information of the subject;
an electronics module (3) configured to process the electrical signal provided by the one or more electrodes (2a, 2b, 2c, 2d), and
electrically conductive tracks (2) electrically connecting said one or more electrodes (2a, 2b, 2c, 2d) to the electronics module (3),
wherein the electronics module (3) is arranged on the second bottom surface (8) of the base structure (1), wherein said one or more electrodes (2a, 2b, 2c, 2d) and the electronics module (3) are arranged on the same side of the base structure (1),
wherein when the wearable sensor device (10) is arranged to the skin surface (100) of the subject, said one or more electrodes (2a, 2b, 2c, 2d) and the electronics module (3) are arranged in-between the subject's skin surface (100) and the base structure (1).

2. The wearable sensor device (10) according to claim 1, wherein the first bottom surface (7) is adapted to extend at least partly along a circumference of the second bottom surface (8), preferably 50% - 100%.

3. The wearable sensor device (10) according to any of claims 1 - 2, wherein the first bottom surface (7) and the second bottom surface (8) are at least partly joined by an inclined surface along which said electrically conductive tracks (2) extends between the first bottom surface (7) and the second bottom surface (8).

4. The wearable sensor device (10) according to any of claims 1 - 3, wherein the base structure (1) is made from a flexible material.

5. The wearable sensor device (10) according to any of claims 1 - 4, wherein the base structure (1) comprises two or more first bottom surface portions (7a, 7b), wherein at least one electrode (2a, 2b, 2c, 2d) is arranged on each of the two or more first bottom surface portions (7a, 7b).

6. The wearable sensor device (10) according to claim 5, comprising two electrodes (2a, 2b; 2c, 2d) arranged on a single first bottom surface portion (7a, 7b).

7. The wearable sensor device (10) according to any of claims 1 - 6, wherein the electrically conductive tracks (2) comprises contact terminals (5) adapted to be in contact with corresponding contact terminals (6) on the electronics module.

8. The wearable sensor device (10) according to any of claims 7, wherein the electronics module (3) is arranged on the contact terminals (5) of the electrically conductive tracks (2).

9. The wearable sensor device (10) according to any of claims 1 - 8, wherein the electronics module (3) comprises one or more sensors (4) arranged such that they face the skin surface (100) when the wearable sensor device (10) is arranged thereto.

10. The wearable sensor device (10) according to claim 9, wherein at least one of the one or more sensors (4) is an optical sensor.

11. The wearable sensor device (10) according to any of claims 1 - 10, wherein a top portion of the electronics module (3) is dome shaped.

12. The wearable sensor device (10) according to any of claims 1 - 11, wherein the electronics module (3) comprises a printed circuit board, sensory electronics and a battery.

13. The wearable sensor device (10) according to any of claims 1 - 12, wherein the wearable sensor device (10) is adapted to be fixed to a skin surface (100) by means of an adhesive.

14. A method (M100) of manufacturing a wearable sensor device (10) according to claim 1 - 13, comprising the steps of
providing (M101) a base structure (1),
arranging (M102) electrically conductive tracks (2) such that they extend from the electrodes (2a, 2b, 2c, 2d) to a second bottom surface (8),
arranging (M103) one or more electrodes (2a, 2b, 2c, 2d) on a first bottom surface (7) of the base structure (1),
wherein the first bottom surface is located closer to a skin surface than the second bottom surface when the wearable sensor device is arranged to a subject's skin,
providing (M104) an electronics module (3) comprising one or more contact terminals (6), and
arranging (M105) the electronics module (3) to the base structure (1) such that the one or more contact terminals (6) on the electronics module (3) are in contact with one or more contact terminals (5) of the electrically conductive tracks (2),
wherein said one or more electrodes (2a, 2b, 2c, 2d) and the electronics module (3) are arranged on the same side of the base structure (1).

15. Use of the wearable sensor device (10) according to any of claims 1 - 13, to measure a bio-impedance signal of a subject.

## Patentansprüche

1. Tragbare Sensorvorrichtung (10) zur Anordnung an einer Hautfläche (100) einer Testperson, die Folgendes umfasst:
eine Basisstruktur (1), die, wenn die tragbare Sensorvorrichtung (10) an einer Hautfläche (100) einer Testperson angeordnet ist, eine erste untere Fläche (7) und eine zweite untere Fläche (8) bereitstellt, wobei die erste untere Fläche (7) sich näher an einer Hautfläche (100) befindet als die zweite untere Fläche (8), wenn die tragbare Sensorvorrichtung (10) an einer Hautfläche (100) einer Testperson angeordnet ist;
eine oder mehrere Elektroden (2a, 2b, 2c, 2d), die an der ersten unteren Fläche (7) der Basisstruktur (1) angeordnet sind und dazu ausgestaltet sind, ein elektrisches Signal bereitzustellen, das Informationen der Testperson enthält;
ein Elektronikmodul (3), das dazu ausgestaltet ist, das von der einen oder den mehreren Elektroden (2a, 2b, 2c, 2d) bereitgestellte elektrische Signal zu verarbeiten, und
elektrisch leitfähige Bahnen (2), welche die eine oder mehreren Elektroden (2a, 2b, 2c, 2d) elektrisch mit dem Elektronikmodul (3) verbinden,
wobei das Elektronikmodul (3) auf der zweiten unteren Fläche (8) der Basisstruktur (1) angeordnet ist, wobei die eine oder mehreren Elektroden (2a, 2b, 2c, 2d) und das Elektronikmodul (3) auf der gleichen Seite der Basisstruktur (1) angeordnet sind,
wobei, wenn die tragbare Sensorvorrichtung (10) an der Hautfläche (100) der Testperson angeordnet ist, die eine oder mehreren Elektroden (2a, 2b, 2c, 2d) und das Elektronikmodul (3) zwischen der Hautfläche (100) der Testpperson und der Basisstruktur (1) angeordnet sind.

2. Tragbare Sensorvorrichtung (10) nach Anspruch 1, wobei die erste untere Fläche (7) angepasst ist, um sich zumindest teilweise entlang eines Umfangs der zweiten unteren Fläche (8), vorzugsweise 50 % bis 100 %, zu erstrecken.

3. Tragbare Sensorvorrichtung (10) nach einem der Ansprüche 1 bis 2, wobei die erste untere Fläche (7) und die zweite untere Fläche (8) zumindest teilweise durch eine geneigte Fläche verbunden sind, entlang der sich die elektrisch leitfähigen Bahnen (2) zwischen der ersten unteren Fläche (7) und der zweiten unteren Fläche (8) erstrecken.

4. Tragbare Sensorvorrichtung (10) nach einem der Ansprüche 1 bis 3, wobei die Basisstruktur (1) aus einem biegsamen Material besteht.

5. Tragbare Sensorvorrichtung (10) nach einem der Ansprüche 1 bis 4, wobei die Basisstruktur (1) zwei oder mehr erste untere Flächenabschnitte (7a, 7b) umfasst, wobei mindestens eine Elektrode (2a, 2b, 2c, 2d) an jedem der zwei oder mehr ersten unteren Flächenabschnitte (7a, 7b) angeordnet ist.

6. Tragbare Sensorvorrichtung (10) nach Anspruch 5, die zwei Elektroden (2a, 2b; 2c, 2d) umfasst, die auf einem einzigen ersten unteren Flächenabschnitt (7a, 7b) angeordnet sind.

7. Tragbare Sensorvorrichtung (10) nach einem der Ansprüche 1 bis 6, wobei die elektrisch leitfähigen Bahnen (2) Kontaktanschlüsse (5) umfassen, die angepasst sind, um mit entsprechenden Kontaktanschlüssen (6) auf dem Elektronikmodul in Kontakt zu sein.

8. Tragbare Sensorvorrichtung (10) nach einem der Ansprüche 1 bis 7, wobei das Elektronikmodul (3) auf den Kontaktanschlüssen (5) der elektrisch leitfähigen Bahnen (2) angeordnet ist.

9. Tragbare Sensorvorrichtung (10) nach einem der Ansprüche 1 bis 8, wobei das Elektronikmodul (3) einen oder mehrere Sensoren (4) umfasst, die derart angeordnet sind, dass sie der Hautfläche (100) zugewandt sind, wenn die tragbare Sensorvorrichtung (10) daran angeordnet ist.

10. Tragbare Sensorvorrichtung (10) nach Anspruch 9, wobei mindestens einer von dem einen oder den mehreren Sensoren (4) ein optischer Sensor ist.

11. Tragbare Sensorvorrichtung (10) nach einem der Ansprüche 1 bis 10, wobei ein oberer Abschnitt des Elektronikmoduls (3) kuppelförmig ist.

12. Tragbare Sensorvorrichtung (10) nach einem der Ansprüche 1 bis 11, wobei das Elektronikmodul (3) eine gedruckte Leiterplatte, Sensorelektronik und eine Batterie umfasst.

13. Tragbare Sensorvorrichtung (10) nach einem der Ansprüche 1 bis 12, wobei die tragbare Sensorvorrichtung (10) angepasst ist, um mittels eines Haftmittels an einer Hautfläche (100) befestigt zu werden.

14. Verfahren (M100) zur Herstellung einer tragbaren Sensorvorrichtung (10) nach Anspruch 1 bis 13, das die folgenden Schritte umfasst:
Bereitstellen (M101) einer Basisstruktur (1),
Anordnen (M102) von elektrisch leitfähigen Bahnen (2), derart dass sie sich von den Elektroden (2a, 2b, 2c, 2d) zu einer zweiten unteren Fläche (8) erstrecken,
Anordnen (M103) von einer oder mehreren Elektroden (2a, 2b, 2c, 2d) auf einer ersten unteren Fläche (7) der Basisstruktur (1),
wobei die erste untere Fläche sich näher an einer Hautfläche befindet als die zweite untere Fläche, wenn die tragbare Sensorvorrichtung an der Haut einer Testperson angeordnet ist,
Bereitstellen (M104) eines Elektronikmoduls (3), das einen oder mehrere Kontaktanschlüsse (6) umfasst, und
Anordnen (M105) des Elektronikmoduls (3) an der Basisstruktur (1), derart dass der eine oder die mehreren Kontaktanschlüsse (6) auf dem Elektronikmodul (3) mit einem oder mehreren Kontaktanschlüssen (5) der elektrisch leitfähigen Bahnen (2) in Kontakt sind,
wobei die eine oder mehreren Elektroden (2a, 2b, 2c, 2d) und das Elektronikmodul (3) auf der gleichen Seite der Basisstruktur (1) angeordnet sind.

15. Verwendung der tragbaren Sensorvorrichtung (10) nach einem der Ansprüche 1 bis 13 zum Messen eines Bioimpedanzsignals einer Testperson.

## Revendications

1. Dispositif de capteur portable (10) destiné à être agencé sur une surface de peau (100) d'un sujet, comprenant
une structure de base (1) qui, lorsque le dispositif de capteur portable (10) est agencé sur une surface de peau (100) d'un sujet, fournit une première surface inférieure (7) et une deuxième surface inférieure (8), dans lequel la première surface inférieure (7) est située plus près d'une surface de peau (100) que la deuxième surface inférieure (8) lorsque le dispositif de capteur portable (10) est agencé sur une surface de peau (100) d'un sujet ;
une ou plusieurs électrodes (2a, 2b, 2c, 2d) agencées sur la première surface inférieure (7) de la structure de base (1) et configurées pour fournir un signal électrique contenant des informations du sujet ;
un module électronique (3) configuré pour traiter le signal électrique fourni par les une ou plusieurs électrodes (2a, 2b, 2c, 2d), et
des pistes électriquement conductrices (2) reliant électriquement lesdites une ou plusieurs électrodes (2a, 2b, 2c, 2d) au module électronique (3),
dans lequel le module électronique (3) est agencé sur la deuxième surface inférieure (8) de la structure de base (1), dans lequel lesdites une ou plusieurs électrodes (2a, 2b, 2c, 2d) et le module électronique (3) sont agencés sur le même côté de la structure de base (1),
dans lequel, lorsque le dispositif de capteur portable (10) est agencé sur la surface de peau (100) du sujet, lesdites une ou plusieurs électrodes (2a, 2b, 2c, 2d) et le module électronique (3) sont agencés entre la surface de peau (100) du sujet et la structure de base (1).

2. Dispositif de capteur portable (10) selon la revendication 1, dans lequel la première surface inférieure (7) est apte à s'étendre au moins partiellement le long d'une circonférence de la deuxième surface inférieure (8) de préférence de 50 % à 100 %.

3. Dispositif de capteur portable (10) selon la revendication 1 ou 2, dans lequel la première surface inférieure (7) et la deuxième surface inférieure (8) sont au moins partiellement jointes par une surface inclinée le long de laquelle lesdites pistes électriquement conductrices (22) s'étendent entre la première surface inférieure (7) et la deuxième surface inférieure (8).

4. Dispositif de capteur portable (10) selon l'une quelconque des revendications 1 à 3, dans lequel
la structure de base (1) est constituée d'un matériau souple.

5. Dispositif de capteur portable (10) selon l'une quelconque des revendications 1 à 4, dans lequel
la structure de base (1) comprend deux premières parties de surface inférieure (7a, 7b) ou plus, dans lequel au moins une électrode (2a, 2b, 2c, 2d) est agencée sur chacune des deux premières parties de surface inférieure (7a, 7b) ou plus.

6. Dispositif de capteur portable (10) selon la revendication 5, comprenant deux électrodes (2a, 2b, 2c, 2d) agencées sur une seule première partie de surface inférieure (7a, 7b).

7. Dispositif de capteur portable (10) selon l'une quelconque des revendications 1 à 6, dans lequel les pistes électriquement conductrices (2) comprennent des bornes de contact (5) aptes à être en contact avec des bornes de contact (6) correspondantes sur le module électronique.

8. Dispositif de capteur portable (10) selon la revendication 7, dans lequel le module électronique (3) est agencé sur les bornes de contact (5) des pistes électriquement conductrices (2).

9. Dispositif de capteur portable (10) selon l'une quelconque des revendications 1 à 8, dans lequel le module électronique (3) comprend un ou plusieurs capteurs (4) agencés de manière à faire face à la surface de peau (100) lorsque le dispositif de capteur portable (10) est agencé sur celle-ci.

10. Dispositif de capteur portable (10) selon la revendication 9, dans lequel au moins l'un des un ou plusieurs capteurs (4) est un capteur optique.

11. Dispositif de capteur portable (10) selon l'une quelconque des revendications 1 à 10, dans lequel une partie supérieure du module électronique (3) est en forme de dôme.

12. Dispositif de capteur portable (10) selon l'une quelconque des revendications 1 à 11, dans lequel le module électronique (3) comprend une carte de circuit imprimé, un circuit électronique sensoriel et une batterie.

13. Dispositif de capteur portable (10) selon l'une quelconque des revendications 1 à 12, dans lequel le dispositif de capteur portable (10) est apte à être fixé à une surface de peau (100) au moyen d'un adhésif.

14. Procédé (M100) de fabrication d'un dispositif de capteur portable (10) selon l'une quelconque des revendications 1 à 13, comprenant les étapes suivantes :
la fourniture (M101) d'une structure de base (1),
l'agencement (M102) de pistes électriquement conductrices (2) de sorte qu'elles s'étendent depuis les électrodes (2a, 2b, 2c, 2d) jusqu'à une deuxième surface inférieure (8),
l'agencement (M103) d'une ou plusieurs électrodes (2a, 2b, 2c, 2d) sur une première surface inférieure (7) de la structure de base (1),
dans lequel la première surface inférieure est située plus près d'une surface de peau que la deuxième surface inférieure lorsque le dispositif de capteur portable est agencé sur la peau d'un sujet,
la fourniture (M104) d'un module électronique (3) comprenant une ou plusieurs bornes de contact (6), et
l'agencement (M105) du module électronique (3) sur la structure de base (1) de sorte que les une ou plusieurs bornes de contact (6) sur le module électronique (3) soient en contact avec une ou plusieurs bornes de contact (5) des pistes électriquement conductrices (2),
dans lequel lesdites une ou plusieurs électrodes (2a, 2b, 2c, 2d) et le module électronique (3) sont agencés sur le même côté de la structure de base (1).

15. Utilisation du dispositif de capteur portable (10) selon l'une quelconque des revendications 1 à 13, pour mesurer un signal de bio-impédance d'un sujet.
